# EUROPEAN PATENT SPECIFICATION

(11) **EP 1 827 546 B1**
(45) Date of publication and mention of the grant of the patent: **10.08.2011**
(21) Application number: 05821209.3
(22) Date of filing: 27.10.2005
(51) Int. Cl.: A61M 25/00

(54) **CATHETER HAVING IMPROVED TORQUE RESPONSE AND CURVE RETENTION**
KATHETER MIT VERBESSERTER DREHMOMENTREAKTION UND KURVEN-RETENTION
CATHETER PRESENTANT UNE REPONSE DE COUPLE ET UNE RETENTION DES COURBES AMELIOREES

(30) Priority: 19.11.2004 US 993586
(43) Date of publication of application: 05.09.2007
(73) Proprietor: Boston Scientific Limited, St. Michael, Barbados, West Indies (BB)
(72) Inventor: ZHOU, Pu, Maple Grove, Minnesota 55311 (US)
(74) Representative: Vossius & Partner
(86) International application number: PCT/US2005/038626
(87) International publication number: WO 2006/055201

(56) References cited:
- WO-A-01/54761
- US-A- 5 769 830
- US-A- 5 772 609
- US-A- 6 143 013

## Description

### Field of the Invention

The invention relates to intracorporeal medical devices, for example, intravascular catheters, and improved methods for manufacturing medical devices. More particularly, the invention relates to methods for manufacturing medical devices that include disposing a braid or braided support structure over a core member. The individual filaments or wires making up the braid may include a section having a non-circular cross-sectional shape and another section having a generally circular cross-sectional shape over the length thereof, as defined in claim 1.

### Background

A wide variety of intracorporeal medical devices have been developed for medical use, for example, intravascular use. Some of these devices include catheters and guidewires that include a braided support structure. These medical devices are manufactured by any one of a variety of different manufacturing methods. Of the known medical device and manufacturing methods, each has certain advantages and disadvantages. There is an ongoing need to provide alternative medical devices and manufacturing methods for producing medical devices with desirable characteristics.

US -A- 5,769,830 discloses a guiding catheter including an atraumatic tip. The guiding catheter includes a reinforcing braid formed from a circular wire. In one embodiment, the circular wire of the braid is ground to form a distal tapered portion of the guiding catheter.

US-A-6,143,013 discloses a catheter with a proximal portion with braided wires with flat cross-section and a distal portion with a separate coil of wire with round cross-section.

### Brief Summary

The invention provides design, material, and manufacturing method alternatives for intracorporeal medical devices such as catheters, guidewires, and the like. In at least some embodiments, the medical devices include a catheter shaft having a braid or support member disposed over at least a portion of the length thereof. The braid is made up of a plurality of wires. At least one of the wires making up the braid includes a section having a non-circular cross-sectional shape and another section having a generally circular cross-sectional shape over the length of the individual filament or wire. The methods for making these types of medical devices may include providing a plurality of wires and altering the cross-sectional shape of a portion of the length of the wires. The wires having the combination of the round shape and altered cross-sectional shape can be formed into a braid and disposed about the core member or formed as a braid onto the shaft.

The above summary of some embodiments is not intended to describe each disclosed embodiment or every implementation of the present invention. The Figures, and Detailed Description, which follow, more particularly exemplify these embodiments.

### Brief Description of the Drawings

The invention may be more completely understood in consideration of the following detailed description of various embodiments of the invention in connection with the accompanying drawings, in which:
Figure 1 is a plan view of an example catheter;
Figure 2 is a partially cut-away view of a portion of the catheter shown in Figure 1;
Figure 3 is a longitudinal cross-sectional view of a portion of the catheter shown in Figures 1 and 2;
Figure 4 is a cross-sectional view of a portion of an example guidewire;
Figure 5 is a plan view of a portion of an example wire;
Figure 6 is a cross-sectional view taken through line 6-6;
Figure 7 is a cross-sectional view taken through line 7-7;
Figure 8 is a perspective view of the wire shown in Figure 5 where the shape of a portion of the wire is altered;
Figure 9 is a cross-sectional view taken through line 9-9;
Figure 10 is an alternative cross-sectional view of an example wire;
Figure 11 is another alternative cross-sectional view of an example wire;
Figure 12 is another alternative cross-sectional an example wire;
Figure 13 is a plan view of a plurality of wires being braided and disposed on a core member; and
Figure 14 is an illustration of another example medical device with an outer layer removed to show the braid configuration.

### Detailed Description

The following description should be read with reference to the drawings wherein like reference numerals indicate like elements throughout the several views. The detailed description and drawings illustrate example embodiments of the claimed invention.

All numeric values are herein assumed to be modified by the term "about," whether or not explicitly indicated. The term "about" generally refers to a range of numbers that one of skill in the art would consider equivalent to the recited value (i.e., having the same function or result). In many instances, the terms "about" may include numbers that are rounded to the nearest significant figure.

The recitation of numerical ranges by endpoints includes all numbers within that range (e.g., 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

The following detailed description should be read with reference to the drawings in which similar elements in different drawings are numbered the same. The drawings, which are not necessarily to scale, depict illustrative embodiments and are not intended to limit the scope of the invention.

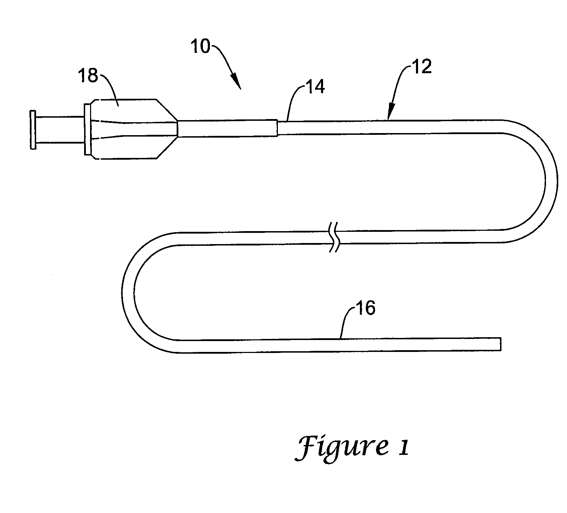

Figure 1 is a plan view of an example medical device depicted as a catheter 10. Catheter 10 may be used for intravascular procedures according to common practice and procedure. For example, catheter 10 may be used to diagnose or treat a medical condition. As such, catheter 10 may be a guide catheter, balloon catheter, cutting balloon catheter, and the like, or any other type of catheter. In addition, catheter 10 may be used in conjunction with or take the form of any other medical device such as a guidewire, endoscopic device, laproscopic device, embolic protection device, and the like, or any other suitable device. Of course, numerous other uses, configurations, and arrangements are known amongst clinicians for catheters and other similarly configured medical devices.

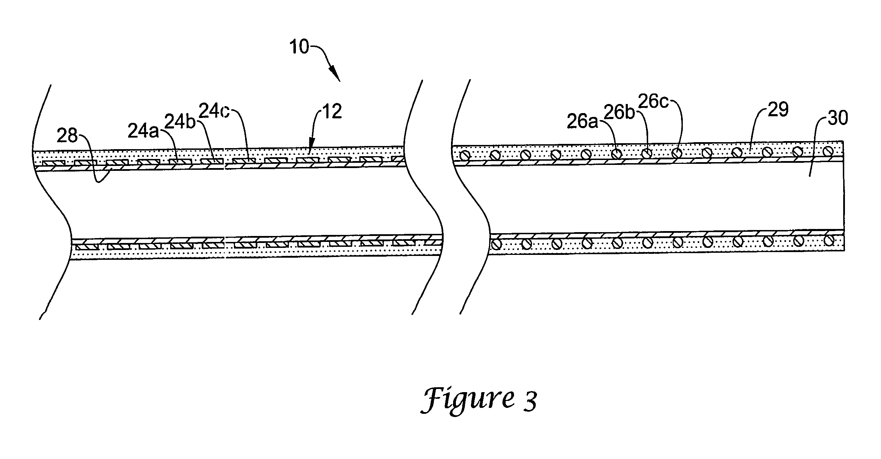

Catheter 10 includes a catheter shaft 12 having a proximal end region 14 and a distal end region 16. A hub or manifold 18 may be disposed adjacent proximal end region 14. One or more lumens (as shown in Figure 3) may be defined in shaft 12 that extend between proximal end region 14 and distal end region 16. In some embodiments, catheter 10 may be a guide catheter. The use of catheter 10 may be similar to the use of typical catheters. For example, catheter 10 may be advanced through the vasculature of a patient to a location adjacent a target region. Catheter 10 may then be used for its intended purpose. For example, if catheter 10 is a guide catheter (as shown) then another diagnostic or therapeutic medical device may be advanced through (i.e., through a lumen defined therein) catheter 10.

A number of support structures are commonly part of a catheter's design. Generally, these support structures provide a particular support feature or features such as torque response, kink resistance, pushability, curve performance, curve support, etc. One such support structure is a braid that may be disposed over a portion or all of the catheter. Braids are typically made from either a flat ribbon-like wire or from a round wire. Flat wires are desirable because they improve the torque response and kink resistance of the catheter. Flat wires, however, tend to provide less desirable curve performance. Round wire, in contrast, provides better curve performance and curve support but less desirable torque response and kink resistance when compared with flat wires. Up until now, catheter designers had to choose between flat wires and round wires when manufacturing catheters that include a continuous braided support structure.

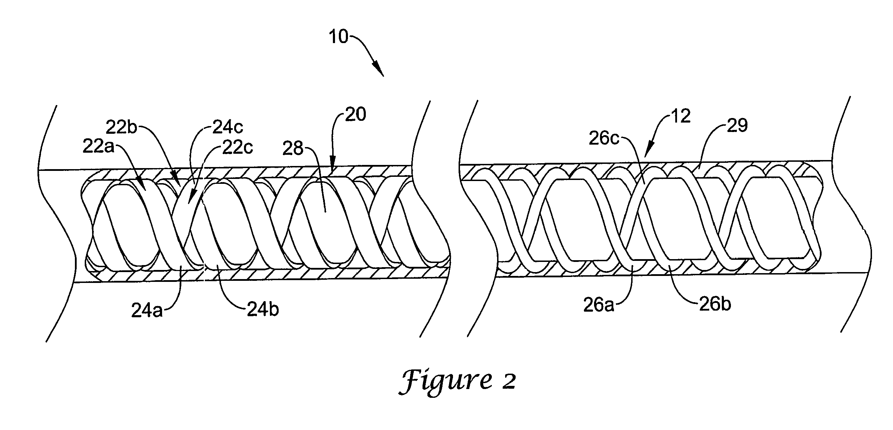

In at least some embodiments, the inventive catheter 10 includes a support structure or braid 20 that has the desirable features of both a flat wire and a round wire as illustrated in Figure 2. For example, braid 20 is made up of a plurality of individual wires 22 (indicated in Figure 2 by reference numbers 22a, 22b, and 22c) that are braided together. In at least some embodiments, each of the wires 22a/22b/22c have a first section 24a/b/c having a non-circular cross-sectional shape and a second section 26a/b/c having a generally circular cross-sectional shape along the individual wires. Other embodiments include only some of the wires 22a/b/c having a non-circular first section 24a/b/c. According to these embodiments, braid 20 includes a mixture of some of wires 22a/b/c having first section 24a/b/c and second section 26a/b/c whereas some of the other wires 22a/b/c may have a constant shape and/or only include differences in diameter.

First sections 24a/b/c and second sections 26a/b/c can be disposed about a core member 28 at the appropriate location so as to impart the desired characteristics to catheter 10. For example, it may be desirable to dispose first sections 24a/b/c near proximal portion 14 of catheter shaft 12 so as to provide a desirable level of proximal torque response. In addition, it may be desirable to dispose second sections 26a/b/c near distal portion 16 of catheter shaft 12 as defined in the claims so as to provide a desirable level of distal curve performance. Of course, the precise positioning of first sections 24a/b/c and second sections 26a/b/c can vary greatly according to claim 1 and can include any position along the length of catheter shaft 12 for either sections 24a/b/c or 26a/b/c.

It should be noted that although Figure 2 depicts wires 22a/b/c having first sections 24a/b/c (as well as second sections 26a/b/c) longitudinally aligned, this need not be the case. Longitudinally aligned is understood to mean that each of the first sections 24a/b/c are located at about the same longitudinal position along shaft 12 and each of the second sections 26a/b/c are located at about the same longitudinal position along shaft 12. Numerous embodiments are contemplated that include non-aligned first sections 24a/b/c and/or second sections 26a/b/c. For example, first section 24a of wire 22a and first section 24b of wire 22b may be longitudinally aligned with second section 26c of wire 22c. Moreover, any of wires 22a/b/c may include multiple first sections 24a/b/c and/or multiple second sections 26a/b/c that can be dispersed anywhere along the length of catheter shaft 12 and may or may not be longitudinally aligned with analogous sections.

Wires 22a/b/c may be made from any suitable material such as a metal, metal alloy, polymer, metal-polymer composite, and the like, or any other suitable material. Some examples of suitable metals and metal alloys include stainless steel, such as 304V, 304L, and 316LV stainless steel; mild steel; nickel-titanium alloy such as linear-elastic or super-elastic nitinol, nickel-chromium alloy, nickel-chromium-iron alloy, cobalt alloy, tungsten or tungsten alloys, MP35-N (having a composition of about 35% Ni, 35% Co, 20% Cr, 9.75% Mo, a maximum 1% Fe, a maximum 1% Ti, a maximum 0.25% C, a maximum 0.15% Mn, and a maximum 0.15% Si), hastelloy, monel 400, inconel 825, or the like; other Co-Cr alloys; platinum enriched stainless steel; or other suitable material.

In some embodiments, wires 22a/b/c may be made from, doped with, or otherwise include a radiopaque material. Radiopaque materials are understood to be materials capable of producing a relatively bright image on a fluoroscopy screen or another imaging technique during a medical procedure. This relatively bright image aids the user of catheter 10 in determining its location. Some examples of radiopaque materials can include, but are not limited to, gold, platinum, molybdenum, palladium, tantalum, tungsten or tungsten alloy, plastic material loaded with a radiopaque filler, and the like.

Wires 22a/b/c, or other portions of catheter 10, may include a sheath or coating such as a hydrophobic, hydrophilic, lubricious, protective, or any other suitable type of coating. For example, shaft 12 may include a sheath 29. Suitable lubricious polymers are well known in the art and may include silicone and the like, hydrophilic polymers such as high-density polyethylene (HDPE), polytetrafluoroethylene (PTFE), polyarylene oxides, polyvinylpyrolidones, polyvinylalcohols, hydroxy alkyl cellulosics, algins, saccharides, caprolactones, and the like, and mixtures and combinations thereof. Hydrophilic polymers may be blended among themselves or with formulated amounts of water insoluble compounds (including some polymers) to yield coatings with suitable lubricity, bonding, and solubility. Some other examples of such coatings and materials and methods used to create such coatings can be found in U.S. Patent Nos. 6,139,510 and 5,772,609.

Figure 3 is a cross-sectional view of catheter 10. Here the non-circular (e.g., flat or ribbon-like) cross-sectional shape of first sections 24a/b/c and the generally circular cross-sectional shape for second sections 26a/b/c can be more clearly seen. Further details regarding the numerous options for shape of first sections 24a/b/c and/or section sections 26a/b/c are discussed in more detail below.

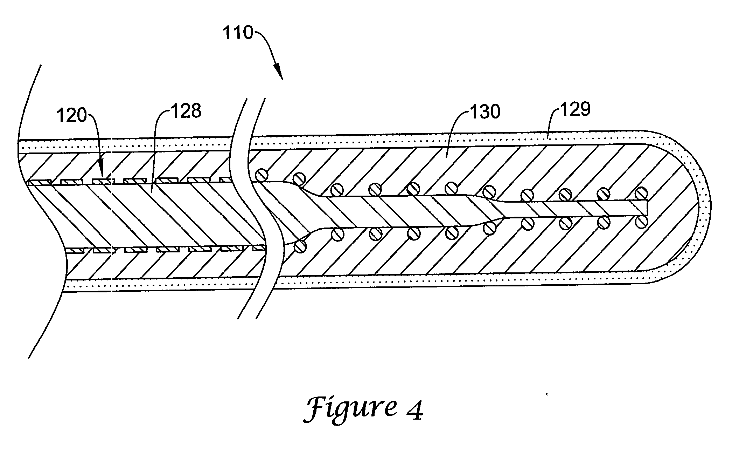

Also seen in Figure 3 is that core member 28 is a catheter core and it includes a lumen 30, for example, a guidewire lumen. As such, this figure is intended to explicitly demonstrate that braid 20 can be used in conjunction with catheters. However, braid 20 is not intended to be limited to just catheters as any suitable medical device may benefit from it design advantages. For example, Figure 4 depicts medical device 110, which takes the form of a guidewire. Guidewire 110 includes core member or core wire 128 having braid 120 disposed thereon. Braid 120 is essentially the same in form and function as braid 20 so that the description of the attributes of braid 20 can be applied to braid 120, to the extent applicable. In some embodiments, guidewire 110 may include a polymer jacket 130 and/or a sheath 129.

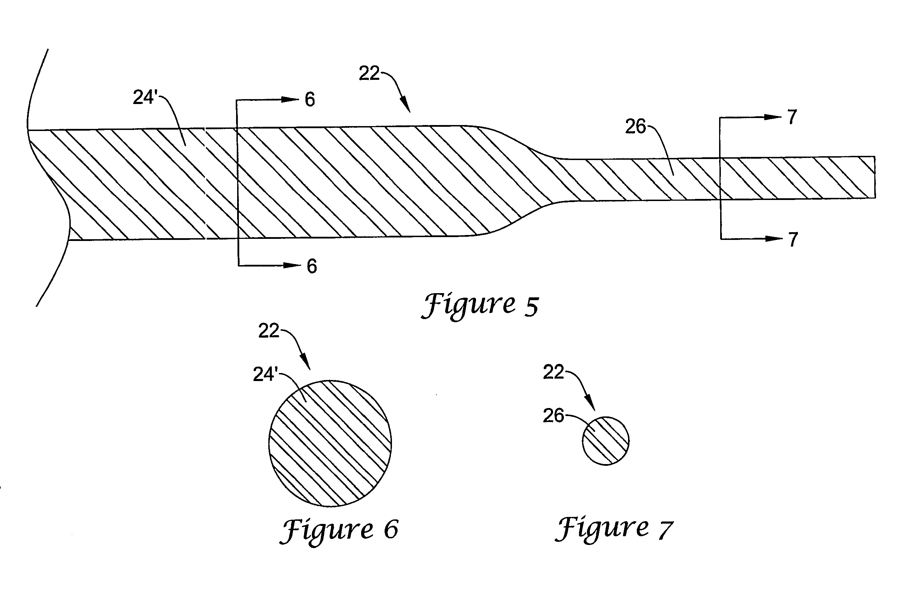

Figures 5-8 illustrate some of the method steps suitable for making catheter 10 or other similarly configured medical devices. Figure 5 depicts wire 22. Wire 22 is similar to other wires used to construct a braid for a medical device. However, wire 22 includes first section 24' (reference number 24' is intended to distinguish this unmodified form of the first section of wire 22 from first section 24) and second section 26. Sections 24'/26, in wire 22 prior to construction, are both generally round and can be distinguished by differences between their respective diameters. For example, by comparing Figure 6 (depicting the diameter of first section 24') with Figure 7 (depicting the diameter of second section 26), it can be seen that second section has a smaller diameter.

The diameter of sections 24'/26 may vary for a given wire. For example, some exemplary wires 22 may include first section 24' with a diameter of about 0.002 to about 0.005 inches and second section 26 with a diameter of about 0.001 to about 0.004 inches. Wires 22 like these are widely available from a number of commercial sources or can be manufactured from commercially available sources of wires or the appropriate starting material. For example, wire 22 can be manufactured by narrowing a portion so as to define second section 26 using known drawing, molding, machining, or similar techniques.

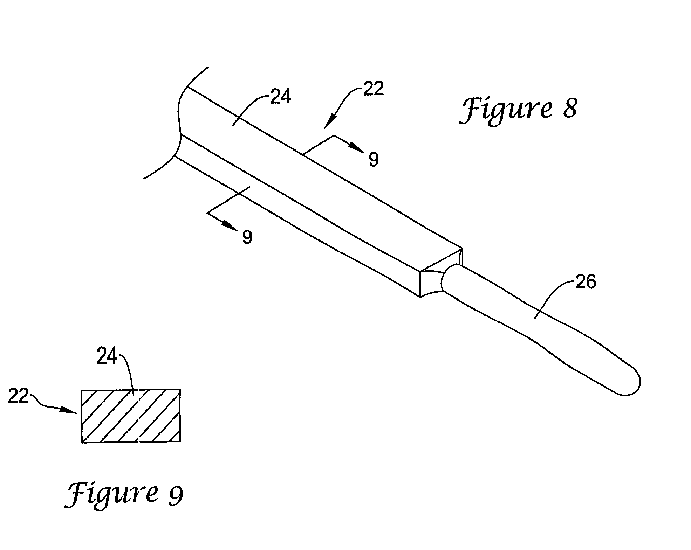

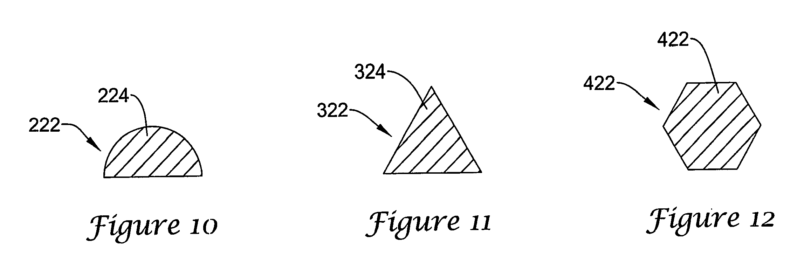

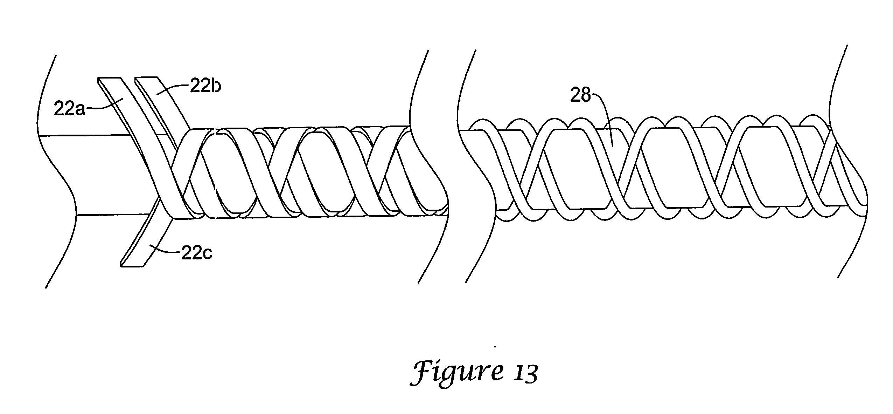

Figure 8 is a perspective view of the wire 22 where first section 24 is altered so as to have a non-circular cross-sectional shape. According to this embodiment, first section 24 is flattened so that it has a rectangular or ribbon-like cross-sectional shape. By altering a portion of wire 22, first section 24 and second section 26 remain continuous with one another. This obviates the need to attempt to attach, weld, or otherwise bond together two dissimilarly shaped wires. As described above, the ribbon-like shape may be desirable for a number of reasons including improved torque response. First section 24, however, is not intended to be limited to precisely this shape because numerous alternative shapes are also contemplated including polygons, ovals, and the like, or any other suitable shape. Figures 10-12 illustrate just a few examples of alternative shapes. For example, Figure 10 illustrates wire 222 having first section 224 that has a semi-circular cross-sectional shape. Figure 11 illustrates wire 322 having first section 324 that has a triangular cross-sectional shape. Figure 12 illustrates wire 422 having first section 424 that has a hexagonal cross-sectional shape. Regardless of which shape first section 24 takes the form of, wires 22a/b/c can be braided about core member 28 as shown in Figure 13. In order to create the desired shape for first section 24 (or any of the alternatives thereof), any suitable alteration technique may be utilized. For example, any suitable stamping, molding, machining, or casting technique can be used.

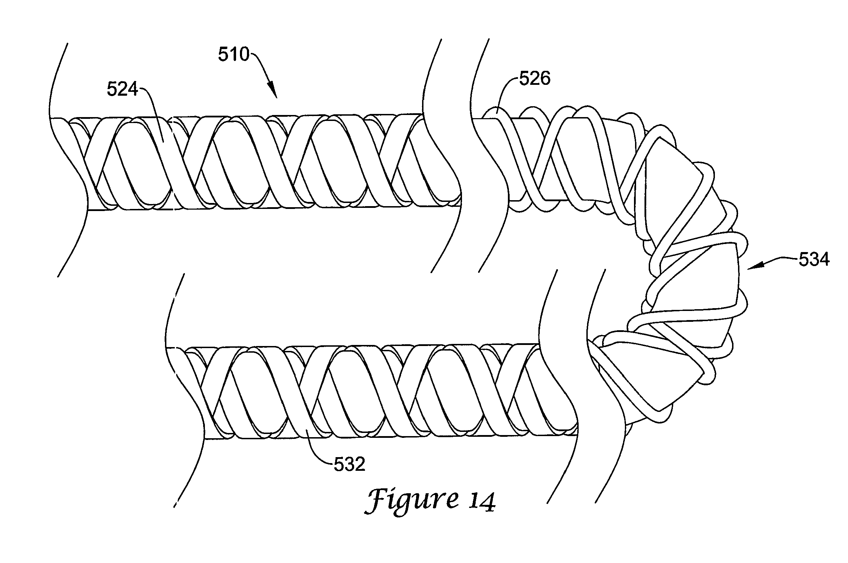

Figure 14 is a partially cut away illustration of another example medical device 510. Medical device 510 is similar to any of the other devices disclosed herein except that in addition to having braid 520 with wires (please note that for clarity purposes the individual wires are not labeled in this drawing) each having first section 524 and second section 526, the wires further include a third section 532. Third section 532, for example, may have a non-circular cross-sectional area. The cross-sectional shape of third section 532 may or may not be the same as first section 524. This embodiment demonstrates that the wires making braid 520 need not be limited to just a single non-circular or to a single generally round section.

Also shown in Figure 14 is an example of the longitudinal and/or spatial distribution of sections 524/526/532 that may be configured to provide device 510 with the desired characteristics. For example, second section 526 is disposed adjacent a curved region 534 of device 510. Because second section 526 includes wires having a generally circular cross-sectional shape, second section 526 can provide a desired level of curve support adjacent curved region 534. In addition, having non-circular first section 524 and third section 532 (which, incidentally, may also be non-circular or generally circular but with, for example, a different diameter than second section 526) allows braid 520 to provide the desired level of torque response as well as the other desirable features of such a configuration.

It should be understood that this disclosure is, in many respects, only illustrative. Changes may be made in details, particularly in matters of shape, size, and arrangement of steps without exceeding the scope of the claims. The invention's scope is, of course, defined in the language in which the appended claims are expressed.

## Claims

1. A medical device including a core member (28) having a proximal section and a distal section, the medical device further comprising:
a plurality of wire filaments including a first wire filament (22a) and a second wire filament (22b) interwoven to form a braid (20) disposed over the core member (28); wherein the first wire filament (22a) includes a proximal portion having a non-circular cross-sectional shape (24a) and a distal portion having a generally circular cross-sectional shape (26a) that is continuous with the proximal non-circular portion (24a).

2. The medical device of claim 1, wherein the second wire filament (22b) includes a first portion with non-circular cross-sectional shape (24b) and a second portion with generally circular cross-sectional shape (26b) that is continuous with the non-circular portion (24b).

3. The medical device of any one of claims 1 or 2, wherein the plurality of wire filaments interwoven to form the braid (20) includes a third wire filament (22c).

4. The medical device of claim 3, wherein the third wire filament (22c) includes a first portion with non-circular cross-sectional shape (24c) and a second portion with generally circular cross-sectional shape (26c) that is continuous with the non-circular portion (24c).

5. The medical device of any one of claims 1, 2, 3 or 4, wherein the non-circular portion (24a) of the first wire filament (22a) is a flattened ribbon portion.

6. The medical device of any one of claims 2, 3, 4 or 5, wherein the non-circular portion (24b) of the second wire filament (22b) is a flattened ribbon portion.

7. The medical device of any one of claims 4, 5 or 6, wherein the non-circular portion (24c) of the third wire filament (22c) is a flattened ribbon portion.

8. The medical device of any one of claims 4, 5, 6 or 7, wherein the non-circular portions (24a, 24b, 24c) of each of the first, second and third wire filaments (22a, 22b, 22c) are disposed adjacent the proximal section of the core member (28).

9. The medical device of any one of claims 4, 5, 6, 7 or 8, wherein the circular portions (26a, 26b, 26c) of each of the first, second and third wire filaments (22a, 22b, 22c) are disposed adjacent the distal section of the core member (28).

10. The medical device of claim 4, wherein each of the first second and third wire filaments (22a, 22b, 22c) includes a third portion with non-circular cross-sectional shape (532), and wherein the second circular portion (526) is located between the first non-circular portion (524) and the third non-circular portion (532).

11. The medical device of claim 10, wherein the second circular portion (526) is located along a curved region (534) of the medical device.

12. The medical device of any one of the previous claims, wherein the medical device is one of a catheter, a guidewire or an endoscope.

13. The medical device of any one of the previous claims, wherein the core member (28) is a guidewire core wire (128).

14. The medical device of any one of the previous claims, wherein the core member (28) includes one or more lumens (30).

15. The medical device of any one of the previous claims, wherein the medical device further includes a sheath (29, 129) disposed over the braid (20).

16. The medical device of claim 15, wherein the sheath (29, 129) comprises a lubricious polymer.

17. A method for manufacturing the medical device of claim 1, the method **characterized by** the steps of:
providing a plurality of round wires (22);
altering the cross-sectional shape of a first section of one or more of the plurality of wires (22), such that the one or more altered wires includes a proximal portion having a non- circular section (24) and a distal portion having a circular section (26) continuous with the non- circular section (24);
providing a core member (28) having a proximal section and a distal section; and
interweaving the plurality of wires (22) including the one or more altered wires about the core member (28) to form a braid (20) disposed about the core member (28).

18. The method of claim 17, wherein the step of altering the cross-sectional shape of a first section of one or more of the plurality of wires (22) includes flattening the first section of the one or more altered wires.

19. The method of claim 17 or 18, further **characterized by** the step of reducing the cross-sectional area of one or more of the plurality of round wires (22) prior to altering the cross-sectional shape of the first section of one or more of the plurality of wires (22).

20. The method of claim 19, wherein the step of reducing the cross-sectional area of one or more of the plurality of round wires (22) includes a drawing step.

21. The method of claim 19, wherein the step of reducing the cross-sectional area of one or more of the plurality of round wires (22) includes a machining step.

22. The method of any one of claims 17, 18 or 19, further **characterized by** the step of positioning the non-circular section (24) of the one or more altered wires along the proximal section of the core wire (28).

23. The method of claim 22, further **characterized by** the step of positioning the circular section (26) of the one or more altered wires along the distal section of the core wire (28).

24. The method of claim 17, wherein the medical device is one of a guidewire, a catheter, or an endoscope.

## Patentansprüche

1. Medizinprodukt mit einem Kernteil (28), das ein proximales Teilstück und ein distales Teilstück hat, wobei das Medizinprodukt ferner aufweist:
mehrere Drahtfilamente mit einem ersten Drahtfilament (22a) und einem zweiten Drahtfilament (22b), die so verwoben sind, dass sie ein Geflecht (20) bilden, das über dem Kernteil (28) angeordnet ist; wobei das erste Drahtfilament (22a) einen proximalen Abschnitt mit einer nicht kreisförmigen Querschnittform (24a) und einen distalen Abschnitt mit einer allgemein kreisförmigen Querschnittform (26a) aufweist, der mit dem proximalen nicht kreisförmigen Abschnitt (24a) zusammenhängt.

2. Medizinprodukt nach Anspruch 1, wobei das zweite Drahtfilament (22b) einen ersten Abschnitt mit einer nicht kreisförmigen Querschnittform (24b) und einen zweiten Abschnitt mit einer allgemein kreisförmigen Querschnittform (26b) aufweist, der mit dem nicht kreisförmigen Abschnitt (24b) zusammenhängt.

3. Medizinprodukt nach Anspruch 1 oder 2, wobei die mehreren Drahtfilamente, die so verwoben sind, dass sie das Geflecht (20) bilden, ein drittes Drahtfilament (22c) aufweisen.

4. Medizinprodukt nach Anspruch 3, wobei das dritte Drahtfilament (22c) einen ersten Abschnitt mit einer nicht kreisförmigen Querschnittform (24c) und einen zweiten Abschnitt mit einer allgemein kreisförmigen Querschnittform (26c) aufweist, der mit dem nicht kreisförmigen Abschnitt (24c) zusammenhängt.

5. Medizinprodukt nach Anspruch 1, 2, 3 oder 4, wobei der nicht kreisförmige Abschnitt (24a) des ersten Drahtfilaments (22a) ein abgeflachter Bandabschnitt ist.

6. Medizinprodukt nach Anspruch 2, 3, 4 oder 5, wobei der nicht kreisförmige Abschnitt (24b) des zweiten Drahtfilaments (22b) ein abgeflachter Bandabschnitt ist.

7. Medizinprodukt nach Anspruch 4, 5 oder 6, wobei der nicht kreisförmige Abschnitt (24c) des dritten Drahtfilaments (22c) ein abgeflachter Bandabschnitt ist.

8. Medizinprodukt nach Anspruch 4, 5, 6 oder 7, wobei die nicht kreisförmigen Abschnitte (24a, 24b, 24c) des ersten, zweiten und dritten Drahtfilaments (22a, 22b, 22c) jeweils benachbart zum proximalen Teilstück des Kernteils (28) angeordnet sind.

9. Medizinprodukt nach Anspruch 4, 5, 6, 7 oder 8, wobei die kreisförmigen Abschnitte (26a, 26b, 26c) des ersten, zweiten und dritten Drahtfilaments (22a, 22b, 22c) jeweils benachbart zum distalen Teilstück des Kernteils (28) angeordnet sind.

10. Medizinprodukt nach Anspruch 4, wobei das erste, zweite und dritte Drahtfilament (22a, 22b, 22c) jeweils einen dritten Abschnitt mit einer nicht kreisförmigen Querschnittform (532) aufweisen und wobei der zweite kreisförmige Abschnitt (526) zwischen dem ersten nicht kreisförmigen Abschnitt (524) und dem dritten nicht kreisförmigen Abschnitt (532) liegt.

11. Medizinprodukt nach Anspruch 10, wobei der zweite kreisförmige Abschnitt (526) entlang einem gekrümmten Bereich (534) des Medizinprodukts liegt.

12. Medizinprodukt nach einem der vorstehenden Ansprüche, wobei das Medizinprodukt ein Katheter, ein Führungsdraht oder ein Endoskop ist.

13. Medizinprodukt nach einem der vorstehenden Ansprüche, wobei das Kernteil (28) ein Kerndraht (128) eines Führungsdrahts ist.

14. Medizinprodukt nach einem der vorstehenden Ansprüche, wobei das Kernteil (28) ein oder mehrere Lumen (30) aufweist.

15. Medizinprodukt nach einem der vorstehenden Ansprüche, wobei das Medizinprodukt ferner eine Hülle (29, 129) aufweist, die über dem Geflecht (20) angeordnet ist.

16. Medizinprodukt nach Anspruch 15, wobei die Hülle (29, 129) ein gleitfähiges Polymer aufweist.

17. Verfahren zur Herstellung des Medizinprodukts nach Anspruch 1, wobei das Verfahren **gekennzeichnet ist durch** die Schritte:
Bereitstellen mehrerer Runddrähte (22);
Ändern der Querschnittform eines ersten Teilstücks eines oder mehrerer der mehreren Drähte (22), so dass der eine oder die mehreren geänderten Drähte einen proximalen Abschnitt mit einem nicht kreisförmigen Schnitt (24) und einen distalen Abschnitt mit einem kreisförmigen Schnitt (26) aufweisen, der mit dem nicht kreisförmigen Schnitt (24) zusammenhängt;
Bereitstellen eines Kernteils (28) mit einem proximalen Teilstück und einem distalen Teilstück; und
Verweben der mehreren Drähte (22) mit dem einen oder den mehreren geänderten Drähten um das Kernteil (28), um ein Geflecht (20) zu bilden, das um das Kernteil (28) angeordnet ist.

18. Verfahren nach Anspruch 17, wobei der Schritt des Änderns der Querschnittform eines ersten Teilstücks eines oder mehrerer der mehreren Drähte (22) Abflachen des ersten Teilstücks des einen oder der mehreren geänderten Drähte aufweist.

19. Verfahren nach Anspruch 17 oder 18, ferner **gekennzeichnet durch** den Schritt des Verkleinerns der Querschnittfläche eines oder mehrerer der mehreren Runddrähte (22) vor Ändern der Querschnittform des ersten Teilstücks eines oder mehrerer der mehreren Drähte (22).

20. Verfahren nach Anspruch 19, wobei der Schritt des Verkleinerns der Querschnittfläche eines oder mehrerer der mehreren Runddrähte (22) einen Ziehschritt aufweist.

21. Verfahren nach Anspruch 19, wobei der Schritt des Verkleinerns der Querschnittfläche eines oder mehrerer der mehreren Runddrähte (22) einen mechanischen Bearbeitungsschritt aufweist.

22. Verfahren nach Anspruch 17, 18 oder 19, ferner **gekennzeichnet durch** den Schritt des Positionierens des nicht kreisförmigen Schnitts (24) des einen oder der mehreren geänderten Drähte entlang dem proximalen Teilstück des Kerndrahts (28).

23. Verfahren nach Anspruch 22, ferner **gekennzeichnet durch** den Schritt des Positionierens des kreisförmigen Schnitts (26) des einen oder der mehreren geänderten Drähte entlang dem distalen Teilstück des Kerndrahts (28).

24. Verfahren nach Anspruch 17, wobei das Medizinprodukt ein Führungsdraht, ein Katheter oder ein Endoskop ist.

## Revendications

1. Dispositif médical comprenant un élément d'armature (28) ayant une section proximale et une section distale, le dispositif médical comprenant en outre :
une pluralité de filaments de fil métallique comprenant un premier filament de fil métallique (22a) et un deuxième filament de fil métallique (22b) entrelacés afin de former une tresse (20) disposée sur l'élément d'armature (28) ; dans lequel le premier filament de fil métallique (22a) comprend une partie proximale ayant une forme de section transversale non circulaire (24a) et une partie distale ayant une forme de section transversale généralement circulaire (26a) qui est continue avec la partie proximale non circulaire (24a).

2. Dispositif médical selon la revendication 1, dans lequel le deuxième filament de fil métallique (22b) comprend une première partie avec une forme de section transversale non circulaire (24b) et une deuxième partie avec une forme de section transversale généralement circulaire (26b) qui est continue avec la partie non circulaire (24b).

3. Dispositif médical selon l'une quelconque des revendications 1 ou 2, dans lequel la pluralité de filaments de fil métallique entrelacés pour former la tresse (20) comprend un troisième filament de fil métallique (22c).

4. Dispositif médical selon la revendication 3, dans lequel le troisième filament de fil métallique (22c) comprend une première partie avec une forme de section transversale non circulaire (24c) et une deuxième partie avec une forme de section transversale généralement circulaire (26c) qui est continue avec la partie non circulaire (24c).

5. Dispositif médical selon l'une quelconque des revendications 1, 2, 3 ou 4, dans lequel la partie non circulaire (24a) du premier filament de fil métallique (22a) est une partie de ruban aplatie.

6. Dispositif médical selon l'une quelconque des revendications 2, 3, 4 ou 5, dans lequel la partie non circulaire (24b) du deuxième filament de fil métallique (22b) est une partie de ruban aplatie.

7. Dispositif médical selon l'une quelconque des revendications 4, 5 ou 6, dans lequel la partie non circulaire (24c) du troisième filament de fil métallique (22c) est une partie de ruban aplatie.

8. Dispositif médical selon l'une quelconque des revendications 4, 5, 6 ou 7, dans lequel les parties non circulaires (24a, 24b, 24c) de chacun des premier, deuxième et troisième filaments de fil métallique (22a, 22b, 22c) sont disposées de manière adjacente à la section proximale de l'élément d'armature (28).

9. Dispositif médical selon l'une quelconque des revendications 4, 5, 6, 7 ou 8, dans lequel les parties circulaires (26a, 26b, 26c) de chacun des premier, deuxième et troisième filaments de fil métallique (22a, 22b, 22c) sont disposées de manière adjacente à la section distale de l'élément d'armature (28).

10. Dispositif médical selon la revendication 4, dans lequel chacun des premier, deuxième et troisième filaments de fil métallique (22a, 22b, 22c) comprend une troisième partie avec une forme de section transversale non circulaire (532), et dans lequel la deuxième partie circulaire (526) est positionnée entre la première partie non circulaire (524) et la troisième non circulaire (532).

11. Dispositif médical selon la revendication 10, dans lequel la deuxième partie circulaire (526) est positionnée le long d'une région incurvée (534) du dispositif médical.

12. Dispositif médical selon l'une quelconque des revendications précédentes, dans lequel le dispositif médical est l'un parmi un cathéter, un fil guide ou un endoscope.

13. Dispositif médical selon l'une quelconque des revendications précédentes, dans lequel l'élément d'armature (28) est un fil d'armature (128) de fil guide.

14. Dispositif médical selon l'une quelconque des revendications précédentes, dans lequel l'élément d'armature (28) comprend une ou plusieurs lumières (30).

15. Dispositif médical selon l'une quelconque des revendications précédentes, dans lequel le dispositif médical comprend en outre une gaine (29, 129) disposée sur la tresse (20).

16. Dispositif médical selon la revendication 15, dans lequel la gaine (29, 129) comprend un polymère lubrifiant.

17. Procédé pour fabriquer le dispositif médical selon la revendication 1, le procédé étant **caractérisé par** les étapes consistant à :
prévoir une pluralité de fils métalliques arrondis (22) ;
modifier la forme de section transversale d'une première section d'un ou plusieurs de la pluralité de fils métalliques (22), de sorte que les un ou plusieurs fils métalliques modifiés comprennent une partie proximale ayant une section non circulaire (24) et une partie distale ayant une section circulaire (26) continue avec la section non circulaire (24) ;
prévoir un élément d'armature (28) ayant une section proximale et une section distale ; et
entrelacer la pluralité de fils métalliques (22) comprenant les un ou plusieurs fils modifiés autour de l'élément d'armature (28) afin de former une tresse (20) disposée autour de l'élément d'armature (28).

18. Procédé selon la revendication 17, dans lequel l'étape consistant à modifier la forme de section transversale d'une première section d'un ou de plusieurs fils de la pluralité de fils métalliques (22) comprend l'étape consistant à aplatir la première section des un ou plusieurs fils métalliques modifiés.

19. Procédé selon la revendication 17 ou 18, **caractérisé en outre par** l'étape consistant à réduire la surface transversale d'un ou de plusieurs de la pluralité de fils métalliques arrondis (22) avant de modifier la forme de section transversale de la première section d'un ou de plusieurs fils de la pluralité de fils métalliques (22).

20. Procédé selon la revendication 19, dans lequel l'étape consistant à réduire la surface de section transversale d'un ou de plusieurs fils de la pluralité de fils métalliques arrondis (22) comprend une étape d'étirage.

21. Procédé selon la revendication 19, dans lequel l'étape consistant à réduire la surface de section transversale d'un ou de plusieurs fils de la pluralité de fils métalliques arrondis (22) comprend une étape d'usinage.

22. Procédé selon l'une quelconque des revendications 17, 18 ou 19, **caractérisé en outre par** l'étape consistant à positionner la section non circulaire (24) des un ou plusieurs fils métalliques modifiés le long de la section proximale du fil métallique d'armature (28).

23. Procédé selon la revendication 22, **caractérisé en outre par** l'étape consistant à positionner la section circulaire (26) des un ou plusieurs fils métalliques modifiés le long de la section distale du fil métallique d'armature (28).

24. Procédé selon la revendication 17, dans lequel le dispositif médical est l'un parmi un fil guide, un cathéter ou un endoscope.
